# EUROPEAN PATENT APPLICATION

(11) **EP 2 047 850 A2**
(43) Date of publication of application: **15.04.2009**
(21) Application number: 08170730.9
(22) Date of filing: 12.04.2006
(51) Int. Cl.: A61K 31/426, A61K 31/167, A61P 33/00, A61P 33/02, A61P 31/12, A61P 31/04, A61P 35/00, A61P 29/00

(54) **Methods for treating diseases through inhibition of the function of molecular chaperones such as protein disulfide isomerases , pharmaceutical compositions comprising them, and screening methods for identifying therapeutic agents**

(30) Priority: 12.04.2005 US 670226 P
(62) Divisional of application: 06749878.2
(71) Applicant: Romark Laboratories, L.C., Tampa, FL 33607 (US)
(72) Inventor: Rossignol, Jean-François, St. Petersburg, FL 33710 (US)
(74) Representative: Warcoin, Jacques

(57) **Abstract**

A method of treating an infectious disease caused by a pathogen comprising administering to a subject in need thereof an effective amount of one or more compounds that inhibit the function of a molecular chaperone, wherein said compound is other than tizoxanide or nitazoxanide.

## Description

The present invention relates to methods for treating diseases through interruption of protein maturation, particularly through inhibition of the function of molecular chaperones, such as protein disulfide isomerases, compounds that inhibit the function of molecular chaperones, pharmaceutical compositions comprising them, and screening methods for identifying therapeutic agents for the treatment of a disease based on inhibiting the function of molecular chaperones.

Molecular chaperones are a diverse group of proteins that oversee the correct intracellular folding and assembly of polypeptides without being components of the final structure. Protein disulfide isomerases (PDIs) are a class of molecular chaperones present in lower organisms that is used to facilitate the ordering of proteins being synthesized and their folding. PDIs are also expressed by human cells where they are involved as molecular chaperones and in folding of proteins. Such lower organisms also may require formation of glycoproteins, such that interference with the glycosylation of expressed proteins may inhibit replication of disease causing lower organisms.

In one embodiment, the present invention relates to a method of treating a disease comprising administering to a subject in need thereof a compound that interrupts protein maturation through interference with the function of a molecular chaperone. Preferably, the method utilizes a compound that inhibits a PDI.

Other embodiments include the following. When a compound inhibits the function of a molecular chaperone involved in the folding or glycosylation of proteins, preferably a PDI in certain extra-cellular pathogens (including protozoans, helminths, bacteria and fungi), or in human cells infected with intracellular pathogens (including protozoans, bacteria, fungi and viruses), the infectious organisms are unable to survive and/or replicate. Surprisingly, compounds that inhibit a molecular chaperone, preferably a PDI, involved in these diseases can be used without exerting significant toxicity to healthy human cells. Thus, the present invention relates to a method of treating infectious diseases caused by extra-cellular or intracellular pathogens comprising administering to a subject in need thereof an effective amount of a compound that inhibits the function of a molecular chaperone, preferably a PDI, wherein said compound is other than tizoxanide or nitazoxanide.

When a molecular chaperone-inhibiting or PDI-inhibiting compound is delivered to cells overexpressing pro-inflammatory cytokines, these cytokines are unable to be folded and processed into active forms, and therefore, inflammatory responses are reduced. Surprisingly, compounds that inhibit the function of a molecular chaperone, preferably a PDI involved in these diseases can be used without exerting significant toxicity to healthy human cells. Thus, the present invention relates to a method of treating an inflammatory disease comprising administering to a subject in need thereof an effective amount of a compound that inhibits the function of a molecular chaperone, preferably a PDI.

When a compound binds to a molecular chaperone, preferably a PDI in human cells, for example liver or thyroid cells, which are being targeted by auto-antibodies to PDI, the autoantibodies are unable to attack the PDI-expressing cells and the inflammatory response is reduced. Diabetes, another autoimmune disease, can be treated in some cases by administering a compound that inhibits a PDI involved in degradation of insulin. Surprisingly, compounds that inhibit a PDI involved in an autoimmune disease can be used without significant toxicity to healthy human cells. Thus, the present invention relates to a method of treating an autoimmune disorder comprising administering to a subject in need thereof an effective amount of a compound that inhibits a molecular chaperone, preferably a PDI.

When a compound interferes with the function of a molecular chaperone, preferably a PDI in a human cancer cell, the cancer cell is unable to replicate efficiently. Surprisingly, compounds that inhibit the function of a molecular chaperone, preferably a PDI, involved in a cancer disease can be used without exerting significant toxicity to healthy human cells. Thus, the present invention relates to a method of treating cancer comprising administering to a subject in need thereof an effective amount of a compound that inhibits the function of a molecular chaperone, preferably a PDI.

In addition, the present invention includes a method of identifying a compound for treating a disease comprising providing a molecular chaperone, preferably a PDI enzyme, from a target organism or cell, formulating a model of the enzyme using computational chemistry modeling techniques well known in the art, and using the modeling software to design compounds that bind more efficiently to a desired molecular chaperone. Thus, the present invention relates to a method of identifying a compound suitable for treating a disease comprising applying computational chemistry to a molecular chaperone, PDI or glycosylating enzyme active site, identifying a compound that is predicted to inhibit a pre-selected molecular chaperone, PDI or glycosylating enzyme that is isolated from a pathogenic organism or animal cell, and optionally further determining biological activity of the compound by testing for activity in one or more cell culture, animal model, or clinical tests.

Another embodiment is a compound identified by the screening method of the preceding embodiment.

Unless otherwise specified, the words "a" or "an" as used herein mean "one or more".

The "subject" to be treated according to the present invention is preferably a human subject, but the term "subject" further includes any animal which may be suffering from (i) a disease in which inhibition of the function of the subject's own molecular chaperone, preferably a PDI enzyme, is beneficial (such as, for example, an inflammatory disease where inhibition of the host's endogenous PDI enzyme will suppress cytokine maturation) or (ii) a disease caused by a pathogen which is susceptible to molecular chaperone, preferably a PDI enzyme inhibition.

The compounds that inhibit the function of a molecular chaperone or PDI, referred to herein as a "PDI inhibitor" or "PDI-inhibiting compound" or "molecular chaperone inhibitor" or "molecular chaperone-inhibiting compound" preferably include a peptide bond. Preferably, the substituents on either side of the peptide bond of the inhibitor serve to stabilize the bond in biological fluids and tissues. One embodiment is a compound that inhibits a molecular chaperone, preferably a PDI, of the formula R1-NHCO-R2, wherein R1 and R2 are independently selected moieties that stabilize the NHCO group.

Preferably R1 and R2 are each a substituted or unsubstituted ring, preferably a heterocyclic group or a carbocyclic group such as an aryl or cycloalkyl group. Preferably, R1 is a heterocyclic ring and R2 is an aryl, optionally substituted by one to three substituents.

Even more preferably, R1 is selected from the group consisting of thiazole and thiadiazole substituted by one to three substituents, and R2 is benzene substituted by one to three substituents.

In another preferred group of compounds, R1 and R2 are both a substituted or unsubstituted benzene ring.

Preferred substituents for R1 and R2 include OH, alkoxy, fluoro, alkyl, ester, and thioalkyl. Preferred substituents include OH, OAc, CH3, CF3, N02, CH2CO2Et, SCH3, Br, and OCH3.

Examples of the heterocyclic group for R1 and R2 include for example, an aromatic heterocyclic group or a saturated or unsaturated non-aromatic heterocyclic group (alicyclic heterocyclic group), which contains, besides carbon atoms, at least one heteroatom(s), preferably 1 to 4 heteroatom(s), more preferably, 1 to 2 heteroatom(s), selected from an oxygen atom, a sulfur atom, and a nitrogen atom.

Examples of the "aromatic heterocyclic group" include an aromatic monocyclic heterocyclic group such as a 5 or 6-membered aromatic monoyclic heterocyclic group (e.g., furyl, thienyl, pyrrolyl, oxazolyl, isooxazolyl, thiazolyl, thiadiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.); an aromatic fused heterocyclic group such as a 8 to 12-membered aromatic fused heterocyclic group (e.g., benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, .alpha.-carbolinyl, .beta.-carbolinyl, .gamma.-carbolinyl, acridinyl, phenoxazinyl, phenothiazinyl, phenazinyl, phenoxathinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridaizinyl); preferably, a heterocyclic group consisting of the above-mentioned 5- or 6-membered aromatic monocyclic heterocyclic group fused with a benzene ring or heterocyclic group consisting of the above-mentioned 5- or 6-membered aromatic monocyclic heterocyclic group fused with the same or different above-mentioned 5- or 6-membered aromatic monocyclic heterocyclic group.

Examples of the "non-aromatic heterocyclic group" include a 3 to 8-membered (preferably 5 or 6-membered) saturated or unsaturated (preferably saturated) non-aromatic heterocyclic group (aliphatic heterocyclic group) such as oxiranyl, azetidinyl, oxetanyl, thiethanyl, pyrrolidinyl, tetrahydrofuryl, thiolanyl, piperidinyl, tetrahydropyranyl, morpholinyl, thiomorpholinyl, piperazinyl.

The present invention further includes a pharmaceutical composition comprising one or more molecular chaperone-inhibiting or PDI-inhibiting compounds in an amount effective to inhibit a molecular chaperone or PDI and one or more pharmaceutically acceptable carriers or diluents. The composition may optionally further include one or more additional therapeutic agents targeting the selected disease.

The present invention also relates to kits for accomplishing such treatment comprising (i) an effective amount of a molecular chaperone or PDI inhbitor, (ii) one or more pharmaceutically acceptable carriers and/or additives, and (iii) instructions for use in treating a disease based on molecular chaperone or PDI inhibition.

As used herein, the phrase "instructions for use" shall mean any FDA-mandated labelling, instructions, or package inserts that relate to the administration of a molecular chaperone or PDI inhibitor for the purpose of treating a disease. For example, instructions for use may include, but are not limited to, indications for the particular disease, identification of specific symptoms of the specific disease that can be ameliorated by a molecular chaperone or PDI inhibitor, and recommended dosage amounts for subjects suffering from the disease. The kit of the present invention further comprises a unit dosage amount of the molecular chaperone or PDI inhibitor effective for the disease in question.

The amount of PDI and/or molecular chaperone inhibitor which is required in a pharmaceutical composition or kit according to the invention to achieve the desired effect will depend on a number of factors, in particular the specific disease application, the nature of the particular compound used, the mode of administration, and the condition of the patient.

In the manufacture of a pharmaceutical composition according to the invention, hereinafter referred to as a "formulation", the PDI and/or molecular chaperone inhibitor is typically admixed with, *inter alia*, an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.05% to 95% by weight of the active compound. One or more PDI and/or molecular chaperone inhibitors, together with one or more additional therapeutic agents selected for the disease in question, may be incorporated in the formulations of the invention, which may be prepared by any of the well known techniques of pharmacy for admixing the components.

In addition to a PDI and/or molecular chaperone inhibitor, other pharmacologically active substances may be present in the formulations of the present invention which are known to be useful for treating the targeted disease. For example, in the case of treating a viral disease, the compounds of the invention may be present in combination with an anti-viral nucleoside analog (such as entecavir) or other known anti-viral agents.

The formulations of the invention include those suitable for oral, inhalation (in solid and liquid forms), rectal, topical, buccal (e.g. sub-lingual), parenteral (e.g. subcutaneous, intramuscular, intradermal, or intravenous) and transdermal administration, although the most suitable route in any given case will depend on the nature and severity of the condition being treated and on the nature of the particular form of molecular chaperone, PDI and/or glycosylating inhibitor which is being used.

Formulations suitable for oral administration may be presented in discrete units, such as capsules, cachets, lozenges, or tablets, each containing a predetermined amount of a PDI and/or molecular chaperone inhibitor or a physiologically acceptable salt or acid derivative thereof; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients).

In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations suitable for buccal (sub-lingual) administration include lozenges comprising a PDI and/or molecular chaperone inhibitor, in a flavored base, usually sucrose and acacia or tragacanth; and pastilles comprising the compound in an inert base such as gelatin and glycerin or sucrose and acacia.

Formulations of the present invention suitable for parenteral administration conveniently comprise sterile aqueous preparations of a PDI and/or molecular chaperone inhibitor, or a physiologically acceptable salt or acid derivative thereof, which preparations are preferably isotonic with the blood of the intended recipient. These preparations are preferably administered intravenously, although administration may also be effected by means of subcutaneous, intramuscular, or intradermal injection. Such preparations may conveniently be prepared by admixing the compound with water or a glycine buffer and rendering the resulting solution sterile and isotonic with the blood.

Formulations suitable for rectal administration are preferably presented as unit dose suppositories. These may be prepared by admixing a PDI and/or molecular chaperone inhibitor with one or more conventional solid carriers, for example, cocoa butter, and then shaping the resulting mixture.

Formulations suitable for topical application to the skin preferably take the form of an ointment, cream, lotion, paste, gel, spray, aerosol, or oil. Carriers which may be used include vaseline, lanoline, polyethylene glycols, alcohols, and combinations of two or more thereof. Formulations for transdermal administration may be delivered by iontophoresis (see, for example, Pharmaceutical Research 3(6), 318, (1986)) and typically take the form of an optionally buffered aqueous solution of a PDI and/or molecular chaperone inhibitor. Suitable formulations comprise citrate or bis/tris buffer (pH 6) or ethanol/water and contain from 0.1 to 0.2M active ingredient.

The present invention is further illustrated by, though in no way limited to, the following examples.

Cell culture studies of the mechanism of action by which tizoxanide suppresses rotavirus infection have been carried out. While tizoxanide did not affect viral transcription, these studies have shown that it selectively affects the synthesis and/or maturation of a single protein, identified as VP7. More particularly, the results of this study showed that tizoxanide prevented VP7 from reaching a stage of maturation that allowed it to be glycosylated, thus preventing the protein from ultimately maturing into a functional protein.
a. Studies have shown that tizoxanide binds to PDI-4 of *Giardia intestinalis* (an extra-cellular protozoan) and that tizoxanide is effective *in vitro* against *Giardia intestinalis.*
b. Studies have shown that tizoxanide binds to PDI isolated from *Neospora caninum* (intracellular protozoan) and is effective *in vitro* against this organism.
c. Studies have shown that tizoxanide and RM-4819 have been shown to prevent maturation of virus protein in rotavirus cell culture.
d. Studies have shown that tizoxanide inhibits secretion of the following pro-inflammatory cytokines: IL-2, IL-4, IL-5, IL-6, IL-8, IL-10, IL-12 and TNF-alpha.
e. In a human subject with persistently elevated liver enzymes due to autoimmune hepatitis, administration of nitazoxanide reduced liver transaminases to normal levels following a 10-day course of treatment administered orally as one nitazoxanide 500 mg twice daily.
f. Studies have shown that nitazoxanide inhibits the replication of human colon cancer cell lines and a variety of other cell lines.
g. Studies in humans have shown that administration of nitazoxanide at a dose of 7.5 mg/kg twice daily by oral route in children with severe rotavirus disease significantly reduces the duration of illness compared to administration of a placebo.
h. Studies in humans have shown that adults with chronic hepatitis B or with chronic hepatitis C can be effectively treated by administering nitazoxanide 500 mg (in tablet form) twice daily for 24 weeks with the patients having undetectable virus DNA or RNA in their serum at the end of treatment.

The following compounds numbered 1-13 in the table below have been synthesized and tested *in vitro* or in cell culture against *Neospora caninum* (a protozoan), para-influenza virus, sendai virus, influenza A virus, and/or rhinovirus. In addition, applicant notes that compounds 12 and 13 have shown good activity against viruses and *Neospora caninum*. In addition, compound 3 was tested in cell culture to determine cytokine suppressing ability, and it showed activity in suppressing cytokines, in particular IL-1β, IL-6 and TNF-alpha. While the activity varies from compound to compound and against different organisms, all compounds showed significant activity.

**Table 1**

| **Number** | **Structure** | | **MW** | **MF** |
|---|---|---|---|---|
| 1 | 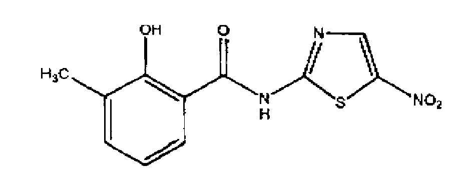 | | 279.27 | C₁₁H₉N₃O₄S |
| 2 | 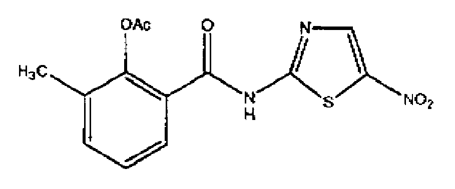 | | 321.31 | C₁₃H₁₁N₃O₅S |
| 3 | 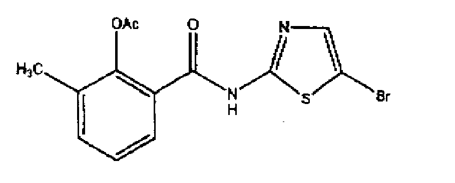 | | 355.21 | C₁₃H₁₁BTN₂O₃S |
| 4 | 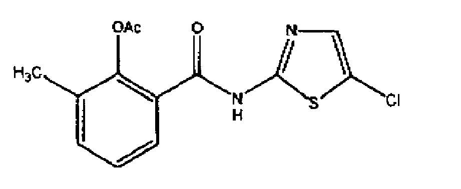 | | 310.76 | C₁₃H₁₁ClN₂O₃S |
| 5 | 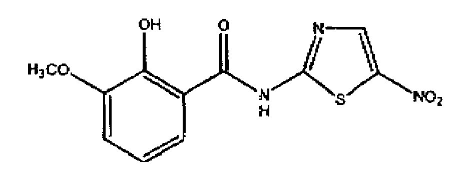 | | 295.27 | C₁₁H₉N₃O₅S |
| 6 | 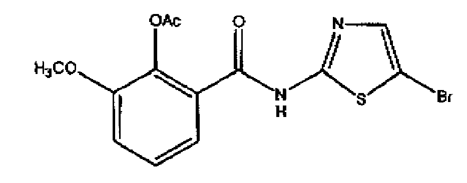 | | 371.21 | C₁₃H₁₁BrN₂O₄S |
| 7 | 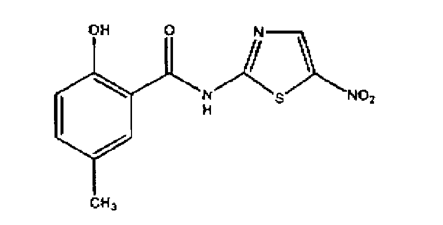 | | 279.27 | C₁₁H₉N₃O₄S |
| 8 | 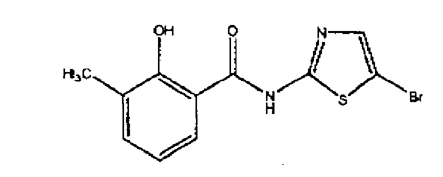 | | 313.17 | C₁₁H₉BrN₂O₂S |
| 9 | 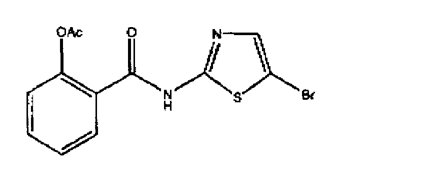 | | 341.18 | C₁₂H₉BrN₂O₃S |
| 10 | 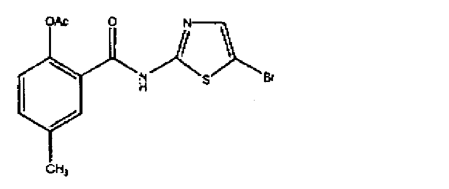 | | 355.21 | C₁₃H₁₁BrN₂O₃S |
| 11 | 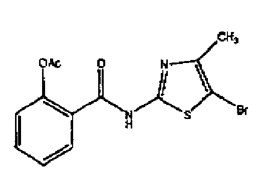 | | 355.21 | C₁₃H₁₁BrN₂O₃S |
| 12 | 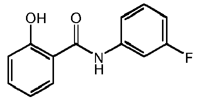 | 231.22 | C₁₃H₁₀FNO₂ | N-(3-fluorophenyl)-2-hydroxybenzamide |
| 13 | 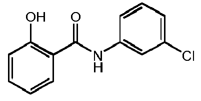 | 247.68 | C₁₃H₁₀ClNO₂ | N-(3-chlorophenyl)-2-hydroxybenzamide |

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

## Claims

1. A compound that inhibits the function of a molecular chaperone, wherein said compound is other than tizoxanide or nitazoxanide for treating an infectious disease caused by a pathogen by administering to a subject in need thereof an effective amount of one or more of said compound.

2. The compound of claim 1 that inhibits the function of a molecular chaperone, wherein the molecular chaperone is a protein disulfide isomerase.

3. The compound of claim 1 or 2 for treating an infectious disease caused by a pathogen, wherein the pathogen is selected from the group consisting of parasites, bacteria, viruses, Neospora caninum, and fungi.

4. The compound of anyone of claims 1 to 3, wherein said compound comprises a peptide bond.

5. The compound of anyone of claim 1 to 4, wherein said compound is represented by the formula R1- NHCO-R2, wherein R1 and R2 are independently selected from moieties that reduce cleavage of the NHCO group in biological fluid and tissue.

6. The compound of claim 5, wherein R1 and R2 are both a substituted or unsubstituted ring.

7. The compound of claim 5 or 6, wherein R1 is a substituted or unsubstituted heterocyclic group and R2 is a substituted or unsubstituted aryl group.

8. The compound of claim 6, wherein R1 is a substituted or unsubstituted thiazole or thiadiazole ring and R2 is a substituted or unsubstituted benzene.

9. The compound of claim 6, wherein R1 and R2 are each substituted with from one to three substituents independently selected from the group consisting of OH, alkoxy, fluoro, alkyl, ester, and thioalkyl.

10. The compound of claim 6, wherein the ring is selected from a heterocyclic group and a carbocyclic group.

11. A compound that inhibits the function of a molecular chaperone for treating an inflammatory disease by administering to a subject in need thereof an effective amount of one or more of said compound.

12. The compound of claim 11 that inhibits the function of a molecular chaperone, wherein the molecular chaperone is a protein disulfide isomerase.

13. A compound that inhibits the function of a molecular chaperone for treating an autoimmune disease by administering to a subject in need thereof an effective amount of one or more of said compound.

14. The compound of claim 13 that inhibits the function of a molecular chaperone, wherein the molecular chaperone is a protein disulfide isomerase.

15. A compound that inhibits the function of a molecular chaperone for treating cancer by administering to a subject in need thereof an effective amount of one or more of said compound.

16. The compound of claim 15 that inhibits the function of a molecular chaperone, wherein the molecular chaperone is a protein disulfide isomerase.

17. A method of identifying a compound suitable for treating a disease comprising applying computational chemistry to an active site of a molecular chaperone isolated from a pathogenic organism or an animal cell, identifying a compound that is predicted to inhibit the active site, and optionally further determining biological activity of the compound by testing for activity in one or more cell culture, animal model, or clinical tests.

18. The method of claim 17, wherein the molecular chaperone is a protein disulfide isornerase.

19. A compound identified by the method of claim 17 for treating a disease by administering to a subject in need thereof said compound.

20. A kit comprising (i) a compound that inhibits a molecular chaperone packaged in one or more unit dosages, wherein each dosage provides an amount effective for inhibiting the function of a molecular chaperone, (ii) a pharmaceutically acceptable carrier or diluent, and instructions for administering the compound under conditions that permit inhibition of the molecular chaperone.

21. The kit of claim 20, wherein the molecular chaperone is a protein disulfide isomerase.

22. The compound of claim 11, wherein the compound is represented by the formula R1 - NHCO-R2, wherein R1 and R2 are independently selected from moieties that reduce cleavage of the NHCO group in biological fluid and tissue.

23. The compound of claim 13, wherein the compound is represented by the formula R1-NT-ICO-R2, wherein R1 and R2 are independently selected from moieties that reduce cleavage of the NHCO group in biological fluid and tissue.

24. The compound of claim 15, wherein the compound is represented by the formula R1 - NIHCO-R2, wherein R1 and R2 are independently selected from moieties that reduce cleavage of the NHCO group in biological fluid and tissue.

25. The compound of claim 6, wherein the compound is selected from the following compounds:
| **Number** | **Structure** | **MW** | **MF** |
|---|---|---|---|
| 1 | 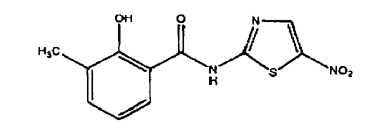 | 279.27 | C₁₁H₉N₃O₄S |
| 2 | 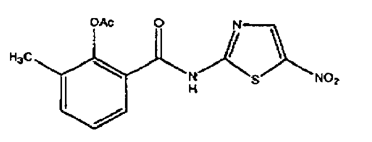 | 321.31 | C₁₃H₁₁N₃O₅S |
| 3 | 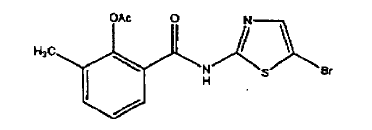 | 355.21 | C₁₃H₁₁BrN₂O₃S |
| 4 | 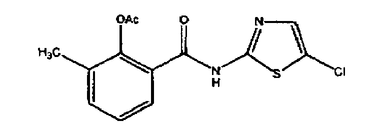 | 310.76 | C₁₃H₁₁ClN₂O₃S |
| 5 | 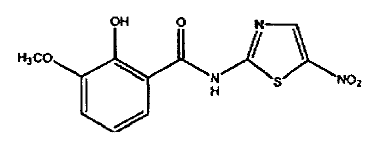 | 295.27 | C₁₁H₉N₃O₅S |
| 6 | 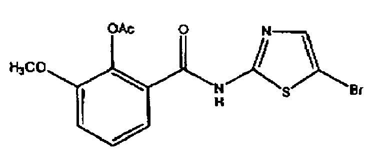 | 371.21 | C₁₃H₁₁BrN₂O₄S |
| 7 | 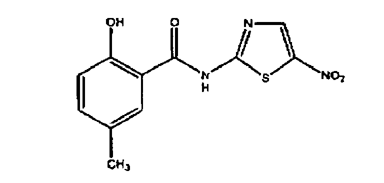 | 279.27 | C₁₁H₉N₃O₄S |
| 8 | 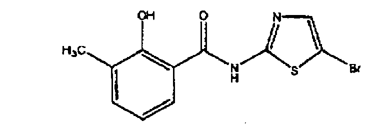 | 313.17 | C₁₁H₉BrN₂O₂S |
| 9 | 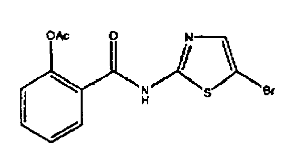 | 341.18 | C₁₂H₉BrN₂O₃S |
| 10 | 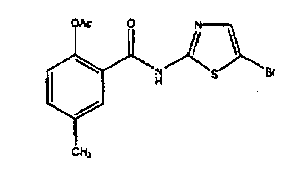 | 355.21 | C₁₃H₁₁BrN₂O₃S |
| 11 | 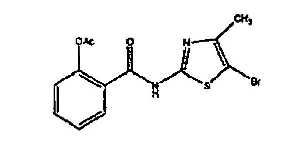 | 355.21 | C₁₃H₁₁BrN₂O₃S |

26. The compound of claim 11, wherein the compound is selected from the following compounds:
| Number | Structure | MW | MF |
|---|---|---|---|
| 1 | 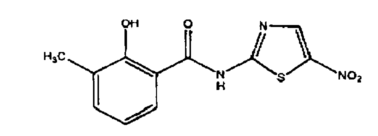 | 279.27 | C₁₁H₉N₃O₄S |
| 2 | 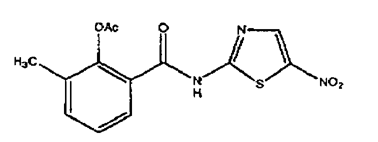 | 321.31 | C₁₃H₁₁N₃O₅S |
| 3 | 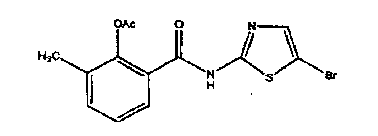 | 355.21 | C₁₃H₁₁BrN₂O₃S |
| 4 | 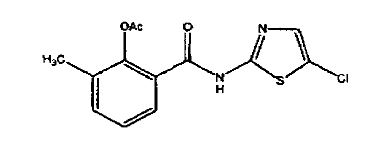 | 310.76 | C₁₃H₁₁ClN₂O₃S |
| 5 | 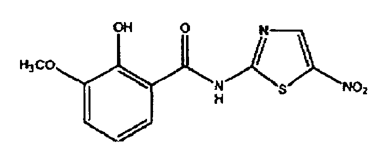 | 295.27 | C₁₁H₉N₃O₅S |
| 6 | 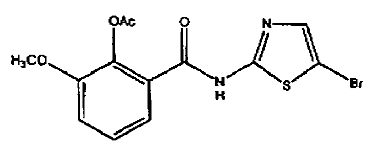 | 371.21 | C₁₃H₁₁BrN₂O₄S |
| 7 | 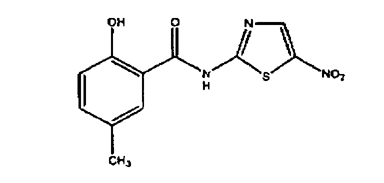 | 279.27 | C₁₁H₉N₃O₄S |
| 8 | 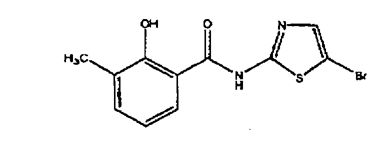 | 313.17 | C₁₁H₉BrN₂O₂S |
| 9 | 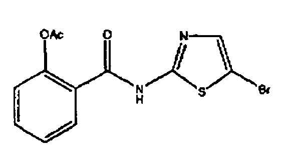 | 341.18 | C₁₂H₉BrN₂O₃S |
| 10 | 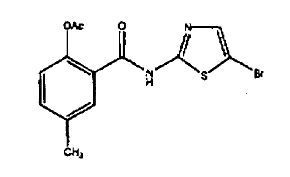 | 355.21 | C₁₃H₁₁BrN₂O₃S |
| 11 | 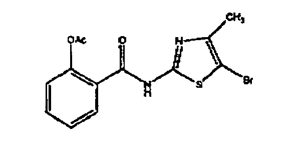 | 355.21 | C₁₃H₁₁BrN₂O₃S |

27. The compound of claim 13, wherein the compound is selected from the following compounds:
| **Number** | **Structure** | **MW** | **MF** |
|---|---|---|---|
| 1 | 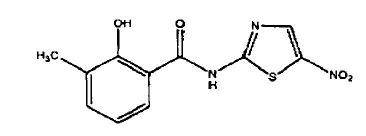 | 279.27 | C₁₁H₉N₃O₄S |
| 2 | 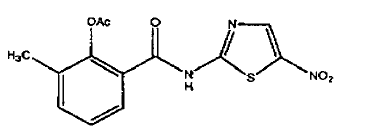 | 321.31 | C₁₃H₁₁N₃O₅S |
| 3 | 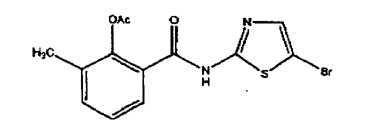 | 355.21 | C₁₃H₁₁BrN₂O₃S |
| 4 | 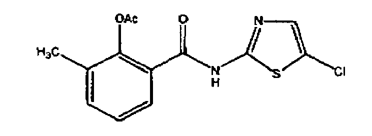 | 310.76 | C₁₃H₁₁ClN₂O₃S |
| 5 | 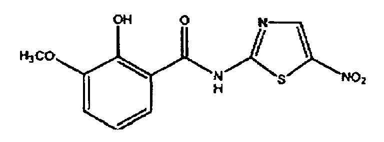 | 295.27 | C₁₁H₉N₃O₅S |
| 6 | 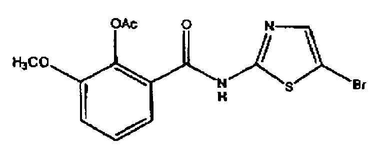 | 371.21 | C₁₃H₁₁BrN₂O₄S |
| 7 | 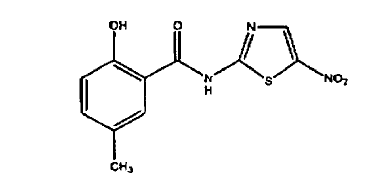 | 279.27 | C₁₁H₉N₃O₄S |
| 8 | 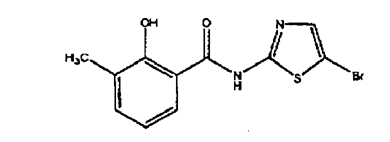 | 313.17 | C₁₁H₉BrN₂O₂S |
| 9 | 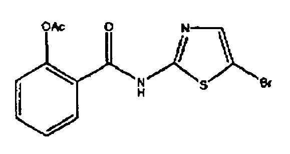 | 341.18 | C₁₂H₉BrN₂O₃S |
| 10 | 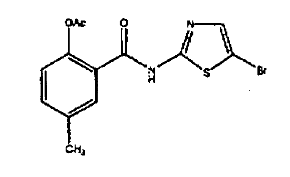 | 355.21 | C₁₃H₁₁BrN₂O₃S |
| 11 | 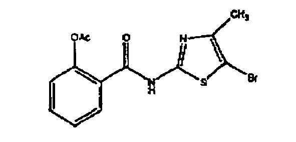 | 355.21 | C₁₃H₁₁BrN₂O₃S |

28. The compound of claim 15, wherein the compound is selected from the following compounds:
| **Number** | **Structure** | **MW** | **MF** |
|---|---|---|---|
| 1 | 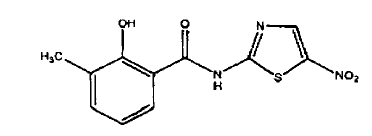 | 279.27 | C₁₁H₉N₃O₄S |
| 2 | 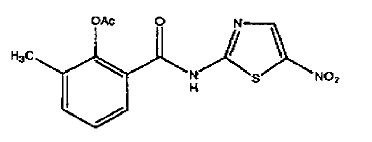 | 321.31 | C₁₃H₁₁N₃O₅S |
| 3 | 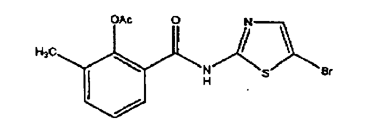 | 355.21 | C₁₃H₁₁rN₂O₃S |
| 4 | 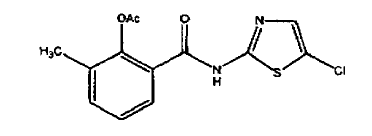 | 310.76 | C₁₃H₁₁ClN₂O₃S |
| 5 | 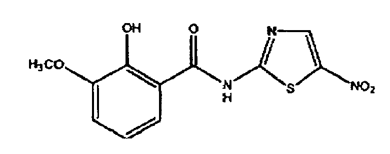 | 295.27 | C₁₁H₉N₃O₅S |
| 6 | 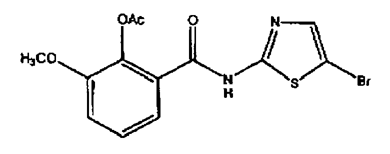 | 371.21 | C₁₃H₁₁BrN₂O₄S |
| 7 | 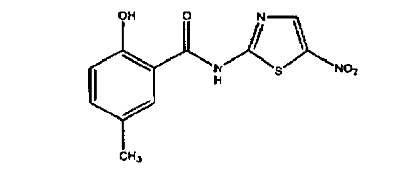 | 279.27 | C₁₁H₉N₃O₄S |
| 8 | 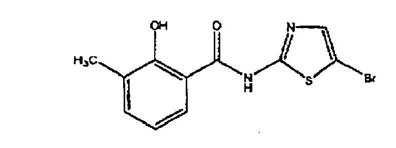 | 313.17 | C₁₁H₉BrN₂O₂S |
| 9 | 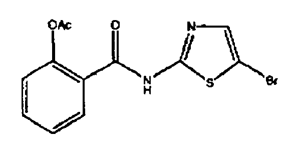 | 341.18 | C₁₂H₉BrN₂O₃S |
| 10 | 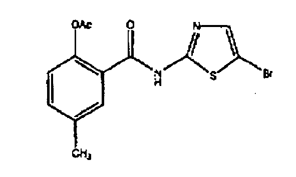 | 355.21 | C₁₃H₁₁BrN₂O₃S |
| 11 | 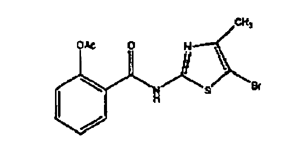 | 355.21 | C₁₃H₁₁BrN₂O₃S |
